# EUROPEAN PATENT APPLICATION

(11) **EP 2 453 242 A1**
(43) Date of publication of application: **16.05.2012**
(21) Application number: 11188751.9
(22) Date of filing: 11.11.2011
(51) Int. Cl.: G01N 33/558, G01N 21/27, G01N 21/93, G01N 35/00

(54) **Method and system utilizing lateral flow immunoassay test device with integrated quality assurance label**

(30) Priority: 12.11.2010 US 413310 P
(71) Applicant: Alere San Diego, Inc., San Diego, CA 92121 (US)
(72) Inventor: Tarpey, Christopher, Los Angeles, CA California 90035 (US)
(74) Representative: Lee, Nicholas John

(57) **Abstract**

The present invention provides a method and system for performing analyte analysis with improved reliability of test results through use of quality assurance labels for accuracy and robust results.

## Description

### CROSS REFERENCE TO RELATED APPLICATIONS

This application claims the benefit of priority under 35 U.S.C. §119(e) of U. S. Serial No. 61/413,310, filed November 12, 2010, the entire contents of which are incorporated herein by reference in its entirety.

### BACKGROUND OF THE INVENTION

### FIELD OF THE INVENTION

The invention provides a method and system for providing quality assurance (QA) confirmation in real-time for solid phase immunoassay test results. The invention is particularly well-suited for use with automated test analysis instrumentation, such as readers and cameras, irrespective of instrument-to-instrument variation in illumination and optical quality.

### BACKGROUND INFORMATION

Immunoassay testing is a promising technology for detecting the presence of an analyte in a sample of biological fluid through the use of immunochemistry. One form of immunoassay testing includes immunochemical based test strips. Conventional test devices include detection zones in which a colored band appears (or is absent) when a specific analyte is present in a human fluid, such as detecting illicit drugs or substances. Conventional test devices provide home testing or convenient clinic-based testing.

However, many tests reveal false positives or false negatives due to a variety of factors, including the inability to discern the intensity of optical density revealed at one or more detection zones of the test device. Coupled with imperfections in manufacturing test strips and challenges in producing easily identifiable results, such errors make use of these strips less than ideal. Incorporation of mechanisms in immunoassay devices to provide quality assurance and reliability of test results would benefit the art.

### SUMMARY OF THE INVENTION

The present invention provides a system and method for performing an immunoassay which utilize a test substrate (such as a nitrocellulose lateral flow immunoassay test strip) providing visually readable test results and having a QA label thereon, which label contains one or more stable optically detectable markers, such as colored lines of known density, adaptable to a plurality of test formats. Methods for use of the test substrates of the invention to improve test result reliability are also provided.

Accordingly, in one aspect, the present invention provides a method of performing an immunoassay. The method includes: a) contacting a lateral flow immunoassay device with a sample, wherein the device includes: i) a detection zone having an analyte binding site; and ii) a QA label having an optically detectable marker of a known density value; b) generating an optical image of the detection zone and the QA label; c) analyzing the image generated in (b) to determine a test density value of the optically detectable marker; d) comparing the test density value with the known density value; and e) analyzing the image generated in (b) to determine a density value of the detection zone when the test density value is within a preset deviation from the known density value.

In another aspect, the present invention provides a system for performing an immunoassay. The system includes: a) a lateral flow immunoassay device including i) a detection zone; and ii) a QA label having at least one optically detectable marker of a known density value; b) an optical image generator; c) a visual display; d) a processor comprising computer-executable instructions for: i) comparing the known density value with a test density value of the at least one optically detectable marker generated via the optical image generator; ii) determining whether the test density value is within a preset deviation of the known density value; and iii) determining the density value of the detection zone when the test density value is within the present deviation; and e) a memory for storage of data relating to one or more immunoassay devices.

### BRIEF DESCRIPTION OF THE DRAWINGS

Figure 1 is a series of schematics illustrating different embodiments of an immunoassay device of the present invention for testing and visually displaying analytical results pertaining to the presence of one or more analytes of interest in a fluid biological sample.

Figure 1A is an immunoassay device in one embodiment of the present invention configured as an integrated cup for detecting presence of seven drugs including methadone (MTD), methylenedioxymethamphetamine (MDMA), methamphetamine (MET), cocaine (COC), marijuana (THC), amphetamine (AMP), and morphine (MOP), as well as conducting Specimen Validity Tests (SVTs) by detection of oxidants (Ox), specific gravity (SG), pH, nitrite (Nit), glutaraldehyde (Glut) and creatine (Cre).

Figure 1B is an immunoassay device in one embodiment of the present invention configured as an integrated cup for detecting presence of eight drugs including AMP, MOP, MET, COC, THC, MTD, MDMA, and buprenorphine (BUP), as well as conducting SVTs by detection of Ox, SG, pH, Nit, Glut and Cre.

Figure 1C is an immunoassay device in one embodiment of the present invention configured as a multi-line device for detecting presence of eight drugs including AMP, MOP, MET, COC, THC, MTD, MDMA and BUP.

Figure 2 provides close-up schematics of an immunoassay device for testing and visually displaying analytical results pertaining to the presence of one or more analytes of interest in a fluid biological sample. Figure 2A is an immunoassay device in one embodiment of the present invention having a QA label **(10).** Figure 2A is an immunoassay device in one embodiment of the present invention having a two dimensional barcode **(20).**

Figure 3 illustrates one embodiment of the system for use with the invention.

Figure 4 is a workflow schematic for performance of an analytical method in one embodiment of the present invention.

Figure 5 shows several QA labels in embodiments of the present invention having optically detectable markers **(30)** present on the QA labels **(10).**

Figure 6 shows a lateral flow immunoassay test device in one embodiment of the present invention including a QA label **(10)** affixed to the top left corner of the device and having an optically detectable markers **(30).**

Figure 7 is a series of schematics illustrating different embodiments of an immunoassay device of the present invention for testing and visually displaying analytical results pertaining to the presence of one or more analytes of interest in a fluid biological sample.

Figure 7A is an immunoassay device in one embodiment of the present invention.

Figure 7B is an immunoassay device in one embodiment of the present invention.

Figure 7C is an immunoassay device in one embodiment of the present invention.

### DETAILED DESCRIPTION OF THE INVENTION

The invention provides an in-test process, high quality QA confirmatory image for accurate analysis and consistent image capture. The invention comprises a test substrate (such as a nitrocellulose lateral flow immunoassay test strip) having a QA label thereon, which label contains at least one optically detectable marker including one or more stable colored lines of known density, adaptable to a plurality of test formats. Using an optical reader as an example of suitable instrumentation for use with the invention, the reader measures the density of this line with every scan and determines whether the density value as determined in a particular scan falls within an acceptable range as compared to the known density value. The QA label simultaneously determines whether critical operating parameters of the reader (such as lamp intensity, scanner calibration, and software algorithms) are performing within specifications. If a reading is outside the accepted range, a test result would be noted as invalid and corrective actions could be implemented.

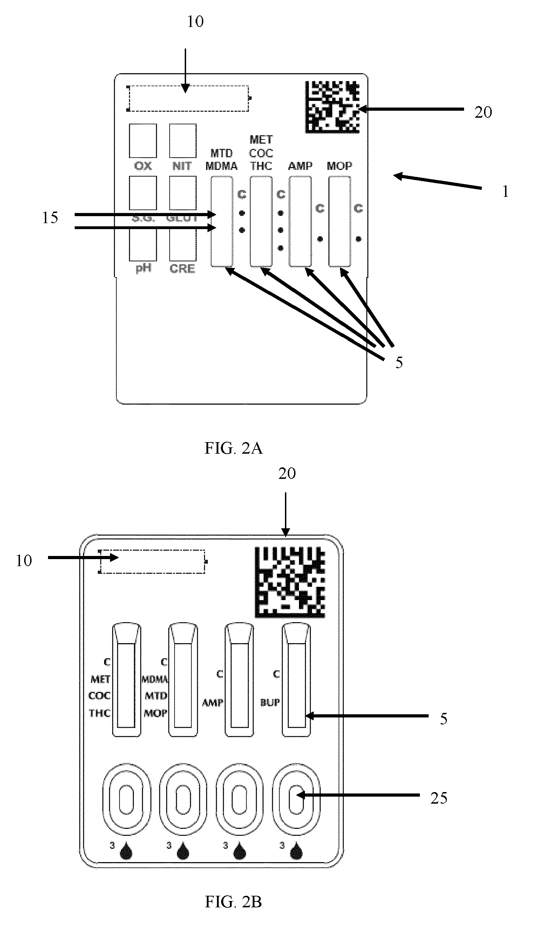

With reference to Figure 2A, in one embodiment, the QA label of the invention is printed or otherwise applied to an immunoassay test device (1) having one or more flow paths **(5)** in fluid communication with at least one or more test or detection zones **(15),** each zone containing an analyte binding site. While the invention will be understood not to be limited to use with lateral flow immunoassay test strip substrate formats, its use can be exemplified by use of such test strips, whose typical configuration is described below.

The use of reagent-impregnated test strips in specific binding assays, such as immunoassays, is well-known (see, e.g., U.S. Patent No. 5,622,871, to May, et al.*,* the text of which is incorporated herein by this reference). With reference to Figure 2A and 2B and by way of illustration, in such procedures, a sample is applied to one portion, e.g., a sample receiving zone **(25),** of the test strip and is allowed to permeate through the strip material. In so doing, the sample progresses from the sample receiving zone **(25)** into or through a detection zone **(15)** in the test strip wherein a specific binding reagent for an analyte suspected of being in the sample is immobilized. Analyte present in the sample can therefore become bound within the detection zone **(15).** The extent to which the analyte becomes bound in that zone can be determined with the aid of labeled reagents which can also be incorporated in the test strip or applied thereto subsequently which allow for optical detection of the labeling reagent.

A typical analytical test device comprises a hollow casing constructed of moisture-impervious solid material (which may be opaque or transparent, but will include visually readable portions at test and control zones) containing a dry porous carrier which communicates directly or indirectly with the exterior of the casing such that a liquid test sample can be applied to the porous carrier. A test device may incorporate a porous solid phase material carrying in a first zone, a labeled reagent which is retained in the first zone while the porous material is in the dry state but may be free to migrate through the porous material when the porous material is moistened, for example by the application of an aqueous liquid sample suspected of containing the analyte to the sample receiving zone **(25).** A second zone, spatially distinct from the first zone, may contain an unlabelled specific binding reagent having specificity for the analyte, and which is capable of participating with the labeled reagent in either a "sandwich" or a "competition" test reaction. Typically, the unlabeled specific binding reagent is immobilized on the porous material at the detection zone **(15)** such that it is not free to migrate when the porous material is in the moist state.

A standard image reader of an optical diagnostic system requires a consistent high quality image for accurate analysis and to ensure a consistent image capture, an in-process quality assurance (QA) system needs to be established. Analyte binding reagents are not present in the QA portion of devices produced and used according to the invention. As discussed above, the present QA approach is to attach a QA label of the present invention, which contains a stable colored line(s) of known density to every test format. As such, conveniently the QA portion of the invention is a pre-printed label including an optically detectable marker of known density, such as a stable colored line(s). A reader measures the density of this line with every scan and determines whether the density value falls within an acceptable range. This ensures that lines on drug panels are being accurately interpreted.

Conveniently, the QA portion of the invention is a label that may be disposed on the test device, adhesively or otherwise. For example, the label may be a pre-printed label formed of a suitable material that may be used in a conventional printing process and affixed to a test device. It will be understood that an optically detectable marker, such as a stable colored line(s) may be printed onto the label via any known printing method, such as digital printing, thermal printing and the like.

In any embodiment of the present invention, the label includes one or more optically detectable markers. For example, a single label may include up to 2, 3, 4, 5, 6, 7, 8, 9, 10 or more optically detectable markers.

As used herein, an "optically detectable marker of known density" is intended to generally refer to a colored feature that is optically detectable and which the optical density is known. As will be appreciated, the term "density" or "optical density" is used synonymously with absorbance, e.g., the ratio of the radiant flux absorbed by a body to that incident upon it, which may be measured over all wavelengths or at one or more specified wavelengths. An optically detectable marker for use with the present invention may be provided in any number of geometric shapes, but generally will be a circle, line, bar, square or ellipse. Additionally, an optically detectable marker may be disposed in or on any region of the QA label.

One of skill in the art would understand that the optically detectable marker may be formed from molecules containing chromophores which may be provided in any form or composition. For example chromophores may be present in any compositions of ink, pigment and the like which produces a consistent marker of known density. Further, the chromophores may be disposed on the QA label, e.g., by printing, stamping or the like, or may be disposed within and throughout the QA label. As such, one skilled in the art would appreciate that chromophores may be incorporated into the QA label when the label is formed.

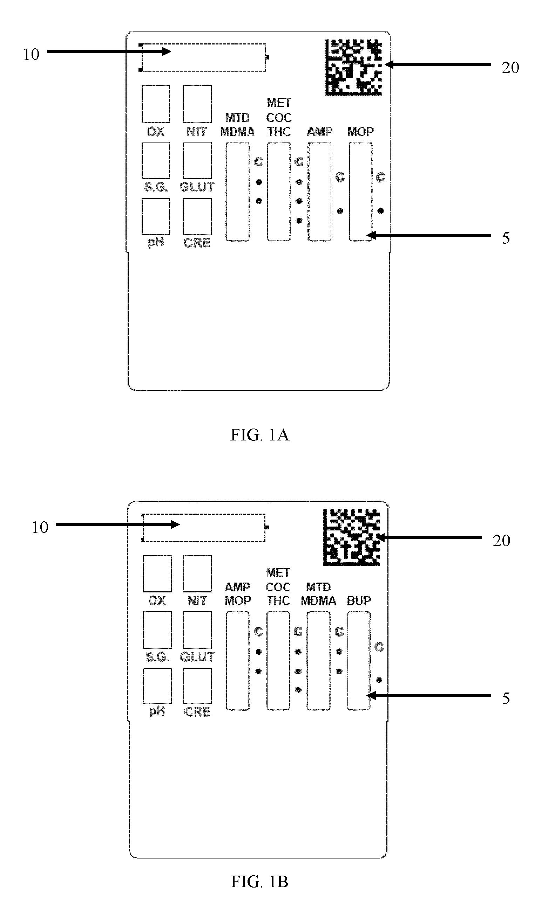

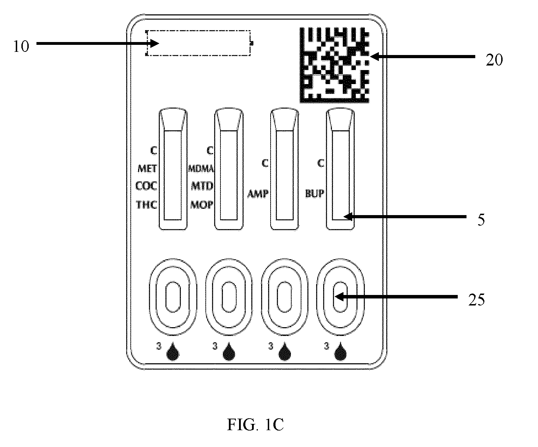

Non-limiting examples of suitable test formats with which a test substrate having the QA features of the invention may be used are illustrated in Figures 1-2 and 5-7.

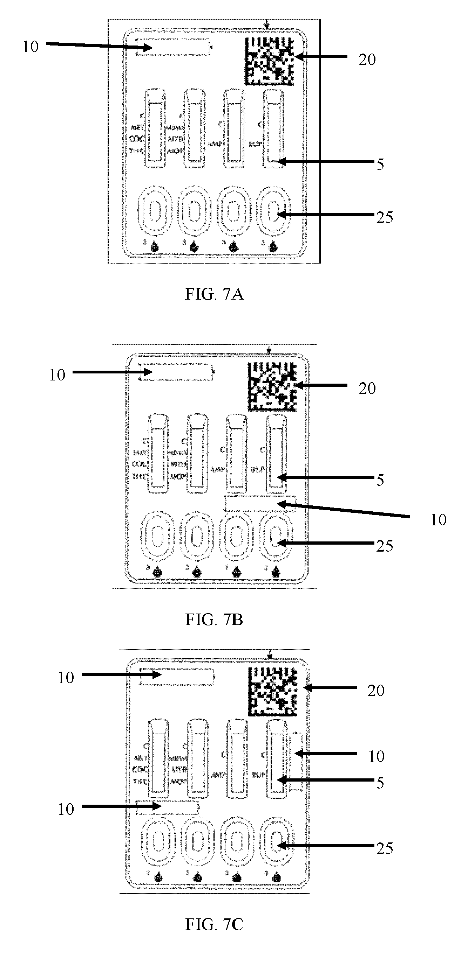

With reference to Figures 7A and 7B, it will be appreciated that a test device may include any number of QA labels disposed at any suitable location on the test device. Figure 7A depicts an embodiment in which a single QA label (**10**) is disposed on the top left corner of the device. Figure 7B depicts an embodiment in which two QA labels **(10)** are disposed on the device and which are spatially separated. Figure 7C depicts an embodiment in which three QA labels **(10)** are disposed on the device and which are spatially separated. As discussed further herein, providing multiple labels at different locations permits greater certainty in the integrity of the light source used to illuminate the device during scanning across the full width of an optical scanning device. As such, in various embodiments, up to 1, 2, 3, 4, 5, 6, 7, 8, 9, 10 or more labels may be provided on the test device. Further, when multiple labels are provided, they may be disposed on opposing edges of the device surface to assist in analyzing scanning across the full width and/or height of the test device.

The immunoassay device of the present invention may also include a barcode. As is generally known in the art, a barcode is an optical machine-readable representation of data. It will also be appreciated that the term barcode is intended to refer to both one dimensional and two dimensional barcodes.

Figures 1, 2, 6 and 7 depict embodiments of the invention in which a two dimensional barcode **(20)** is incorporated. The device may include any number of barcodes disposed at any location suitable for optical recognition. Further, the barcode may include a variety of different data types and assay parameters, such as assay data, detection zone data, calibration data, QA label data, optical data and subject data.

As used herein, "assay data" is intended to refer to data pertaining to the features of the test device and assay(s) being performed on the test device. For example, such data includes the types of assays being performed on the device; analytes detectable using the device; location of physical features of the device, such as geometry of the device, location of QA labels, location of detection and control zones; type and location of SVTs; and device data, such as manufacturing information, age, size, type, and shape of device.

As used herein, "detection zone data" is intended to refer to data pertaining to the detection zones of the device **(15)**. For example, such data includes number, size and location of detection zones; expected color and intensity of label accumulation; and type of label(s).

As used herein, "calibration data" is intended to refer to data pertaining to calibration of the scanning device and optimizing results of image recognition. For example, such data includes density values for positive and negative tests; parameters for increasing or decreasing detected density values; parameters for accepting or dismissing scans; and parameters for assessing light intensity.

As used herein, "QA label data" is intended to refer to data pertaining to the QA label on the test device. For example, such data includes number, shape, size and location of labels; number, shape, size and location of optically detectable markers; and known optical densities of markers.

As used herein, "optical data" is intended to refer to data pertaining to the optical recognition device. For example, such data includes optical recognition device data, such as type, size and recognition software; and scan data, such as wavelength(s) of electromagnetic energy used to perform a scan and scan settings.

As used herein, " subj ect data" is intended to refer to data pertaining to the subj ect from which the analyzed sample is taken. For example, such data includes subject information, such as name, medical history and prior testing data.

Instrumentation with which the QA label of the invention may be used are, as noted, image recognition devices, such as optical readers, scanners, imagers, cameras and other visually responsive analysis instruments. As is appreciated by one in the art, image recognition devices further include smartphones as well as other mobile technology.

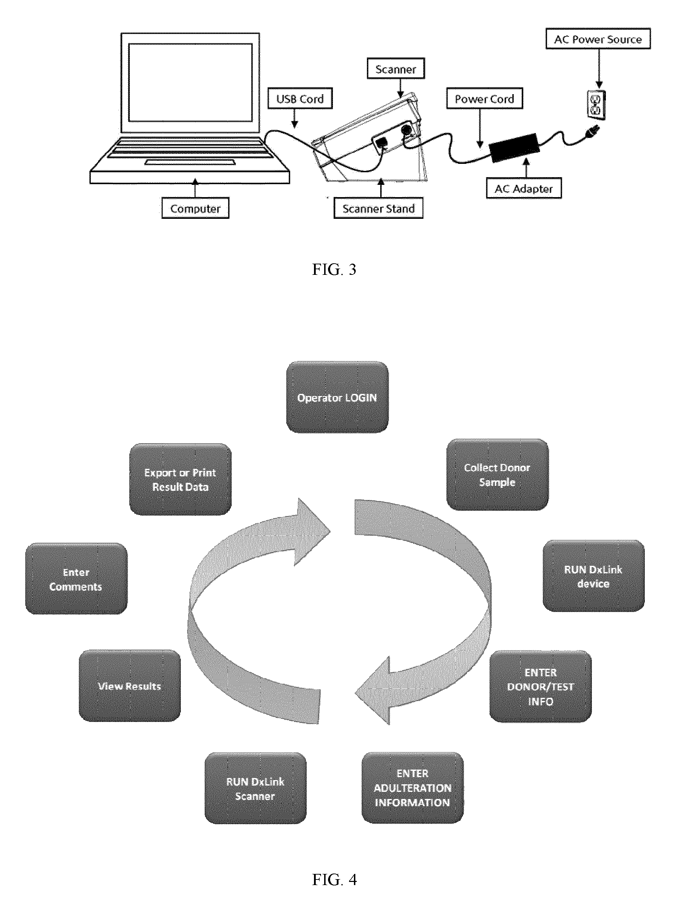

A particularly preferred device for use with the invention is shown in Figure 3. In various aspects of the invention, the device may typically include a scanner or imaging device, a computer having at least one system processor with image processing capability, a computer monitor, an input device, and a power supply. Thus the apparatus includes a computer having executable code for performing the various analyses required to practice the invention.

In various embodiments the system and functionality of the system allow for a variety of beneficial features. Such features include, but are not limited to automated analysis of test panels; automatic test menu recognition (*e.g*., barcode); in-process system quality control checks; image capture, data management/export functionality, and data archiving of results; and validation for use with multiple formats/test menus.

A number of benefits are achieved by the features of the system. Automation of the system eliminates result interpretation variability and subjectivity. Accuracy of the system is far superior than visual interpretation of test panels using the "naked" eye. Automation also allows increased throughput and scanning process speeds of approximately 15 - 30 sec. The flexibility of the system is also paramount and allows for multiple test formats.

In one embodiment, the system for use with the invention is the SureStep™ Instrument Test System with DxLINK™ Technology sold by GenPrime / Innovacon. Alternative commercially available instruments with which the invention may be adapted to use include, without limitation, those sold by Syntron, Mavand, Express Diagnostics, Ultimed, and MedTox.

In use, a method for confirming the validity of a visually readable analyte test result according to the invention is summarized as follows. An operator logs into the computer system. A collected donor sample is applied to a test device, which is then placed in the optical scanner. The software functionality (DxLINK™) is then activated by the operator and any information regarding the donor or test sample is inputted. Next the test device is scanned by activating the scanner. Results may then be viewed and any comments entered. The results may be printed and/or exported and archived in a data storage medium. The method is summarized in Figure 4. Examples of the invention with an optical reader system are provided in Example 1 below.

In one exemplary embodiment, such as that depicted in Figures 1, 2, 6 and 7A, the immunoassay device of the present invention is provided with a single QA label **(10)** of known color intensity (for example, standard RGB or Pantone colors). When the device is scanned, scanning software recognizes the spatial location of the QA label **(10)** and compares the color intensity, e.g., the optical density value, to the expected color intensity of a visually detectable marker disposed on the label.

In other exemplary embodiments, such as those depicted in Figures 7B and 7C, the immunoassay device of the present invention is provided with two or more QA labels **(10)** at different locations on the device. The inclusion of labels **(10)** at more than one location permits greater certainty in the integrity of the light source used to illuminate the device during scanning across the full width of the scanner. Any deviations observed in the acquired image could indicate deterioration in the scan head/light source suggesting that system maintenance is required in order to ensure the integrity of any results obtained from the scanned lateral flow strips.

In other exemplary embodiments, such as those depicted in Figures 1, 2, 6 and 7, to the immunoassay device of the present invention is provided with one or more QA labels **(10)** on the surface of the device. A two dimensional barcode **(20)** is also provided on the device. As discussed above, the barcode includes information related to the known optical density value(s) or color value(s) of markers **(30)** included within the applied QA label(s) **(10)** on the device. When the scanner analyzes a device, the system software analyzes the optical density, e.g., color/intensity of the image in the location of the QA label(s) **(10)** and compares this with the data acquired from the two dimensional barcode **(20).** If the signal from the QA labels **(10)** deviates by more than a predefined value from the parameter stored in the two dimensional barcode **(20),** the result may be rejected and the user prompted to recalibrate or service the scanner.

If the signal from the QA label **(10)** deviates within tolerance limits of the predefined value from the parameter stored in the two dimensional barcode **(20),** in one instance the test result may be automatically adjusted upward or downward according to the scanned value of the optically detectable marker **(30)** of the QA label **(10);** in another instance no change to the signal from the test line is necessary.

As such, the system of the present invention includes a processor having computer-executable instructions for performing such operations. For example, such operations include comparing the known density value with a test density value of at least one optically detectable marker generated via an optical image generator; determining whether the test density value is within a preset deviation of the known density value of an optically detectable marker **(30)** of the QA label **(10);** determining the density value of the detection zone **(15)** and when the test density value is within a preset deviation; increasing (or decreasing) the density value of the detection zone **(15)** when the density value of the optically detectable marker **(30)** is in agreement within error of the defined density value of the QA label **(10).**

In various embodiments, the preset deviation is two, three, four or more standard deviations from the known density value. For example, the preset deviation may be two, three, four or more standard deviations above or below the known density value of the optically detectable marker **(30).**

The following examples are intended to illustrate but not limit the invention.

### EXAMPLE 1

### EVALUATION OF QA LABELS

The GenPrime Reader^{™} consists of an Avision AVA6^{™} flat bed scanner connected to a PC with GenPrime's software algorithm installed. The scanner captures an image of a Drugs-of-Abuse (DOA) test device and the algorithm quantitatively determines whether the presence or absence of a test line is associated with a positive or negative drug result. The Reader requires a consistent high quality image for accurate analysis and to ensure a consistent image capture, an in-process QA system needs to be established. This example describes the use of a QA label, which contains a stable colored line(s) of known density. The Reader measures the density of this line with every scan and determines whether the density value falls within an acceptable range. The analysis of the QA label simultaneously determines whether critical parameters of the Reader such as lamp intensity, scanner calibration, and software algorithms were performing within specifications. This ensures that lines on drug panels were being accurately interpreted. If a reading is outside the accepted range, a drug test result would be noted as invalid and corrective actions could be implemented.

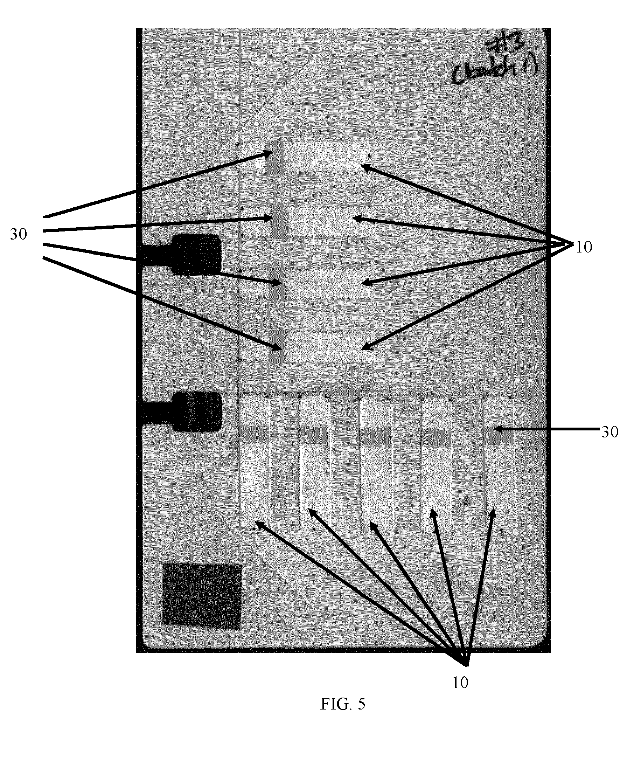

In order to test the concept of an in-process QA system using labels attached to test devices, the consistency of labels manufactured by Typolac were evaluated. These labels are shown in Figure 5. They consist of a 2 mm x 5 mm color stripe on a white 5 mm x 22 mm adhesive label. The label would be affixed to a designated area on the face of the drug of abuse test as shown in Figure 5.

The goal of this study was to evaluate the consistency of the QA label manufactured by Typolac and define the specifications for the QA label. This example describes and discusses the results of these studies.

**Methods**

Evaluation #1: QA Label Variation on Card Surface

In order to determine the level of variation between QA labels, a series of 6.5 cm x 10 cm white index cards were affixed with nine to ten QA labels (Figure 5). Each index card was scanned twelve consecutive times. A total of 672 data points were generated from the scans of 56 total QA labels, which were obtained randomly from various sample sets off the printing reel. From those 672 data points, mean, standard deviation, and coefficient of variation were determined. Figure 5 is a scanned image of the 6.5 cm x 10 cm white index card with nine QA labels attached. There were 9 cards prepared with a total of 56 labels and each card was scanned 12 times for the analysis.

Evaluation #2: QA Label Variation Inside of Key Cup^{™}

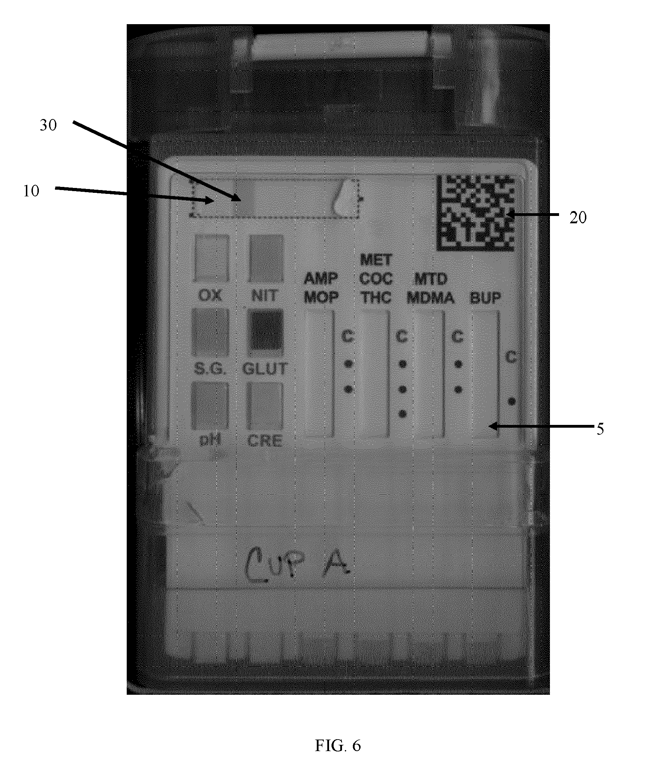

In test formats such as the Key Cup^{™}, the QA labels will be placed under the cup's plastic face. To evaluate the labels' performance under these conditions three DBT-187 Key Cups^{™} were disassembled and the QA label placed in its designated area under the plastic (see Figure 6). Each cup was scanned ten times and a total of 60 data points were assessed. The mean density, standard deviation, and the coefficient of variation were determined. Figure 6 shows an image of a DBT-187 Key Cup^{™} with QA label affixed in its designated area in the upper left corner.

**Results**

Table 1 shows the results of multiple scans of individual labels placed on the index cards. The mean label density ranges between 19.7 and 28.0. The scanner CVs rarely exceed 3%, demonstrating the consistency of the software and scanner system. It was also noted in this study that there was no correlation of mean density to location of the label on the card or whether it was in a vertical or horizontal position. Table 2 displays the mean density for the 56 labels shown in Table 1, which is 23.6 with a standard deviation of 1.7. A value of 23.6 is approximately equivalent to a value somewhere between a G7 and a G8 score on the Abon Gold Color Card^{™} scanned on the surface of a test format. See Table 5 for a comparison of G score values and density measurements. The coefficient of variation for all labels is 7.2%, a measure of label to label variation plus Reader variation, which is expectedly higher than the variation for multiple scans of the same label. One could estimate that the label to label variation is approximately equivalent to the total variation from Table 2 minus the average Reader variation from Table 1 (7.2% - 1.8% = 5.4%). On the other hand, it could be argued that a better measure would be to subtract the maximum Reader variation from the combined variation, which would be 7.2% - 5.8% = 1.4%.

Table 3 displays the results of the analysis of individual Typolac labels placed under the plastic of a Key Cup^{™} as shown in Figure 6. It should be noted that the labels were placed in the designated area where the final QA labels would be attached in the final product. Each data point in Table 3 is a separate scan and it is clear that consistency is not affected for labels placed under the plastic of the Key Cup^{™}. A summary of these data are shown in Table 4. The mean density for labels is 14.7 with a standard deviation of 0.7. The coefficient of variation for labels under these conditions was 4.7%. Also, in this case, a 14.7 density value is approximately equivalent to a G8 score. The reason for the reduced density under these conditions is due to the light absorbing and light scattering properties of the plastic housing.

**Table 1: Mean Density of Individual Labels Placed on Index Cards.**

| Label Number | Mean | Standard Deviation | CV | Label Number | Mean | Standard Deviation | CV |
|---|---|---|---|---|---|---|---|
| 1 | 26.5 | 0.3 | 1.0 | 29 | 19.7 | 0.3 | 1.6 |
| 2 | 24.7 | 0.3 | 1.1 | 30 | 23.9 | 0.4 | 1.8 |
| 3 | 24.6 | 0.3 | 1.4 | 31 | 25.0 | 1.0 | 4.1 |
| 4 | 21.8 | 0.3 | 1.4 | 32 | 25.2 | 0.6 | 2.2 |
| 5 | 25.7 | 0.2 | 0.8 | 33 | 25.7 | 0.2 | 0.9 |
| 6 | 28.0 | 0.5 | 1.9 | 34 | 24.8 | 0.4 | 1.6 |
| 7 | 25.3 | 0.3 | 1.2 | 35 | 24.8 | 0.3 | 1.0 |
| 8 | 21.9 | 0.4 | 1.9 | 36 | 22.7 | 0.5 | 2.1 |
| 9 | 23.5 | 0.5 | 1.9 | 37 | 24.6 | 0.4 | 1.5 |
| 10 | 21.0 | 0.3 | 1.5 | 38 | 24.8 | 0.9 | 3.5 |
| 11 | 23.9 | 0.4 | 1.5 | 39 | 22.6 | 0.3 | 1.3 |
| 12 | 27.2 | 0.4 | 1.6 | 40 | 22.0 | 0.5 | 2.1 |
| 13 | 23.8 | 0.4 | 1.7 | 41 | 23.6 | 0.3 | 1.3 |
| 14 | 22.5 | 0.2 | 1.0 | 42 | 22.3 | 0.2 | 1.1 |
| 15 | 23.7 | 0.5 | 2.0 | 43 | 22.7 | 0.7 | 3.1 |
| 16 | 23.7 | 0.9 | 3.7 | 44 | 25.0 | 0.5 | 1.8 |
| 17 | 23.7 | 0.4 | 1.5 | 45 | 23.8 | 1.4 | 5.8 |
| 18 | 26.3 | 0.2 | 0.8 | 46 | 21.0 | 0.4 | 2.0 |
| 19 | 24.0 | 0.6 | 2.6 | 47 | 23.6 | 0.4 | 1.5 |
| 20 | 20.7 | 0.2 | 1.2 | 48 | 20.8 | 0.3 | 1.5 |
| 21 | 22.7 | 0.4 | 1.7 | 49 | 23.2 | 0.3 | 1.1 |
| 22 | 23.6 | 0.4 | 1.6 | 50 | 22.2 | 0.4 | 2.0 |
| 23 | 20.8 | 0.2 | 0.8 | 51 | 22.7 | 0.4 | 1.8 |
| 24 | 23.3 | 0.3 | 1.1 | 52 | 23.3 | 0.6 | 2.4 |
| 25 | 23.6 | 0.4 | 1.8 | 53 | 24.2 | 0.7 | 2.8 |
| 26 | 23.7 | 0.3 | 1.3 | 54 | 23.8 | 0.5 | 1.9 |
| 27 | 25.0 | 0.3 | 1.1 | 55 | 23.5 | 0.5 | 2.0 |
| 28 | 24.8 | 1.1 | 4.5 | 56 | 22.4 | 0.7 | 2.9 |

**Table 2: Mean Density of All Labels Placed on Index Cards.**

| | |
|---|---|
| Mean Density | 23.6 |
| Standard Deviation | 1.7 |
| Coefficient of Variation | 7.2 |
| Two Standard Deviation Spread | 20.2 - 27.0 |
| Minimum Value | 19.7 |
| Maximum Value | 28.0 |

**Table 3: Density of Individual Labels Placed Inside Key Cup™.**

| Image ID | Scan Number | Density Value | Image ID | Scan Number | Density Value |
|---|---|---|---|---|---|
| Label 1 - Cup A | 1 | 14.8 | Label 4 - Cup A | 1 | 15.3 |
| Label 1 - Cup A | 2 | 14.4 | Label 4 - Cup A | 2 | 15.4 |
| Label 1 - Cup A | 3 | 14.6 | Label 4 - Cup A | 3 | 15.5 |
| Label 1 - Cup A | 4 | 14.5 | Label 4 - Cup A | 4 | 15.5 |
| Label 1 - Cup A | 5 | 14.5 | Label 4 - Cup A | 5 | 15.6 |
| Label 1 - Cup A | 6 | 14.4 | Label 4 - Cup A | 6 | 15.5 |
| Label 1 - Cup A | 7 | 14.4 | Label 4 - Cup A | 7 | 15.7 |
| Label 1 - Cup A | 8 | 14.6 | Label 4 - Cup A | 8 | 15.4 |
| Label 1 - Cup A | 9 | 14.8 | Label 4 - Cup A | 9 | 15.3 |
| Label 1 - Cup A | 10 | 14.0 | Label 4 - Cup A | 10 | 15.6 |
| Label 2 - Cup B | 1 | 15.2 | Label 5 - Cup B | 1 | 11.9 |
| Label 2 - Cup B | 2 | 14.9 | Label 5 - Cup B | 2 | 13.7 |
| Label 2 - Cup B | 3 | 15.1 | Label 5 - Cup B | 3 | 14.2 |
| Label 2 - Cup B | 4 | 15.1 | Label 5 - Cup B | 4 | 15.5 |
| Label 2 - Cup B | 5 | 14.9 | Label 5 - Cup B | 5 | 15.8 |
| Label 2 - Cup B | 6 | 15.2 | Label 5 - Cup B | 6 | 15.7 |
| Label 2 - Cup B | 7 | 15.1 | Label 5 - Cup B | 7 | 15.6 |
| Label 2 - Cup B | 8 | 15.0 | Label 5 - Cup B | 8 | 15.8 |
| Label 2 - Cup B | 9 | 15.0 | Label 5 - Cup B | 9 | 15.7 |
| Label 2 - Cup B | 10 | 15.2 | Label 5 - Cup B | 10 | 14.4 |
| Label 3 - Cup C | 1 | 13.9 | Label 6 - Cup C | 1 | 14.9 |
| Label 3 - Cup C | 2 | 14.2 | Label 6 - Cup C | 2 | 14.3 |
| Label 3 - Cup C | 3 | 14.1 | Label 6 - Cup C | 3 | 14.6 |
| Label 3 - Cup C | 4 | 13.9 | Label 6 - Cup C | 4 | 14.7 |
| Label 3 - Cup C | 5 | 13.8 | Label 6 - Cup C | 5 | 14.6 |
| Lab el 3 - Cup C | 6 | 14.0 | Label 6 - Cup C | 6 | 14.4 |
| Label 3 - Cup C | 7 | 13.9 | Label 6 - Cup C | 7 | 14.8 |
| Label 3 - Cup C | 8 | 14.5 | Label 6 - Cup C | 8 | 14.6 |
| Label 3 - Cup C | 9 | 13.9 | Label 6 - Cup C | 9 | 14.7 |
| Label 3 - Cup C | 10 | 14.0 | Label 6 - Cup C | 10 | 14.5 |

**Table 4: Mean Density of All Labels Placed Inside Key Cup^{™}.**

| | |
|---|---|
| Mean Density | 14.7 |
| Standard Deviation | 0.7 |
| Coefficient of Variation | 4.7 |
| Two Standard Deviation Spread | 13.3 - 16.1 |
| Minimum | 11.9 |
| Maximum | 15.8 |

**Table 5. Mean Density Values for G Lines and QA Labels.**

| | G9 | G8 | G7 | G6 | Mean QA Label Value |
|---|---|---|---|---|---|
| Directly on scanner surface | 34.1 | 26.6 | 19.9 | 16.5 | 23.6 |
| Within Key Cup^{™} under plastic face | 21.3 | 13.2 | 8.6 | 5.9 | 14.7 |

**Conclusions**

The purpose of this study was to evaluate the printing consistency of the Typolac QA label and generate quantitative data that would permit GenPrime to set specifications for the label. There were three major evaluations in this study. The first involved assessing the variation from multiple scans of a single label (n=12 scans). This provided data on the consistency of the Reader, which is a measure of both image capture and software algorithm variation. The second experiment evaluated the label to label variability from randomly selected labels. This tested the printing consistency of the labels plus the Reader variation and allowed determination of a QA specification for labels on the Multi-line card format. The third experiment determined the value and variability of labels placed under the plastic cover of a Key Cup^{™} test format. This tested how the plastic barrier affects the magnitude of both label density and variability.

It should be noted that three different variability measurements were generated in this study. The first shows the intra-label variation of 12 separate scans of a single label, which encompasses the combined variation of image capture and software analysis. These data are displayed in Table 1. The second, which is shown in Table 2, illustrates the total label to label variation of 56 individual labels, which takes into account both printing variation and Reader variation. The third, shown in Table 4 displays the label to label variation for labels placed under the plastic housing of a Key Cup^{™}.

Thus, data are presented showing Reader variation and label to label variation. The latter includes results for both a multi line card device and under the plastic of a Key Cup^{™} device. These exemplify two types of formats in which QA labels may be applied.

It was found that the Reader variation was relatively small. The CV rarely exceeded 3%. The QA label brightness of labels on the index cards is estimated to equate to a density of somewhere between G7 and G8 and has a mean density of 23.6 with an overall coefficient of variation of 7.2% for all labels when placed on the surface of a card.

The mean density for labels under the Key Cup^{™} plastic housing was 14.7 with a coefficient of variation of 4.7%. This value more closely equates to a G8 score. Note in Table 5 that a G8 has a density of 26.6 on the surface of the card and a density of 13.2 under the plastic housing.

For labels placed on the card surface, the minimum and maximum values were 19.7 and 28.0 respectively. For labels in the Key Cup^{™}, the minimum and maximum values were 11.9 and 15.8 respectively. These minimum and maximum values were to set acceptable ranges for the QA label at time of manufacture. Alternatively, the values may be set to an acceptable range to be within two standard deviations from the mean of these values. This specification would represent 95% of all theoretical values for an infinite sample size.

The range for a QA label on the surface of a Multi-line format test device may be set to between 20.2 and 26.8 density values. For labels under the plastic housing of Key Cup^{™}, the range for a QA label on the surface of a Multi-line format should be set between 13.3 and 16.1 density values. Test formats with labels falling outside this range would be rejected as invalid.

It is contemplated that future versions of the QA label may contain at least two color strips, one of G6 and one of G4. This would serve not only to survey image quality but also image sensitivity. An exemplary QA label would have a density of at least one line in the range that would allow accurate interpretation of G4 values.

Although the invention has been described with reference to the above example, it will be understood that modifications and variations are encompassed within the spirit and scope of the invention. Accordingly, the invention is limited only by the following claims.

## Claims

1. A method of performing an immunoassay comprising:
(a) contacting a lateral flow immunoassay device with a sample, wherein the device comprises:
(i) a detection zone having an analyte binding site; and
(ii) a quality assurance (QA) label having an optically detectable marker of a known density value;
(b) generating an optical image of the detection zone and the QA label;
(c) analyzing the image generated in (b) to determine a test density value of the optically detectable marker;
(d) comparing the test density value with the known density value; and
(e) analyzing the image generated in (b) to determine a density value of the detection zone when the test density value is within a preset deviation from the known density value.

2. The method of claim 1, wherein the preset deviation is two, three, four or more standard deviations from the known density value.

3. The method of claim 1, wherein the QA label comprises a plurality of optically detectable markers of known density values.

4. The method of claim 3, wherein the known density value for each optically detectable marker is the same or different.

5. The method of claim 1, wherein the device comprise at least two QA labels, each comprising at least one optically detectable marker of a known density value.

6. The method of claim 1, wherein the QA labels are disposed at different locations on the device.

7. The method of claim 5, wherein the known density value of each optically detectable marker is the same or different.

8. The method of claim 1, wherein the device further comprises a barcode, the barcode comprising data of an assay parameter selected from assay data, detection zone data, calibration data, QA label data, optical data, subject data.

9. The method of claim 8, wherein the barcode is one-dimensional or two-dimensional.

10. The method of claim 1, further comprising increasing the density value of the detection zone where the density value of the optically detectable marker is below the known density value.

11. A system for performing an immunoassay comprising:
a) a lateral flow immunoassay device comprising:
(i) a detection zone; and
(ii) a QA label having at least one optically detectable marker of a known density value;
b) an optical image generator;
c) a visual display;
d) a processor comprising computer-executable instructions for:
(i) comparing the known density value with a test density value of the at least one optically detectable marker generated via the optical image generator;
(ii) determining whether the test density value is within a preset deviation of the known density value; and
(iii) determining the density value of the detection zone when the test density value is within the preset deviation; and
e) a memory for storage of data relating to one or more immunoassay devices.

12. The system of claim 13, wherein the preset deviation is two, three, four or more standard deviations from the known density value.

13. The system of claim 13, wherein the device further comprises a barcode comprising data of an assay parameter selected from assay data, detection zone data, calibration data, QA label data, optical data, subject data.

14. The system of claim 11, wherein the processor further comprises computer-executable instructions for increasing the density value of the detection zone where the density value of the optically detectable marker is below the known density value.

15. The system of claim 11, wherein the device comprise at least two QA labels, each comprising at least one optically detectable marker of a known density value and optionally disposed at different locations on the device.
